# EUROPÄISCHE PATENTANMELDUNG

(11) **EP 2 546 202 A1**
(43) Veröffentlichungstag der Anmeldung: **16.01.2013**
(21) Anmeldenummer: 12169634.8
(22) Anmeldetag: 05.07.2008
(51) Int. Cl.: C02F 1/461, C11D 7/10, C11D 3/48, A61K 33/04, A61K 33/06

(54) **Verwendung von Diaphragmalyselösungen zur Entkeimung von Oberflächen**

(30) Priorität: 18.07.2007 DE 102007033445
(62) Teilanmeldung aus: 08773877.9
(71) Anmelder: Monopharm Handelsgesellschaft Mbh, 21444 Vierhöfen (DE)
(72) Erfinder: Ponitz, Burkhard, 21444 Vierhöfen (DE)
(74) Vertreter: von Puttkamer · Berngruber

(57) **Zusammenfassung**

Verwendung einer Katholyt-Lösung, die in einem Diaphragmalyse-Verfahren erhalten wird. Der Anodenraum und der Kathodenraum einer Elektrolysezelle, sind durch ein Diaphragma voneinander getrennt.

Bei dem Verfahren wird jeweils eine wässrige Alkalichlorid-Lösung zugeführt zur Entkeimung und/oder Desinfektion von Oberflächen.

## Beschreibung

Die Erfindung bezieht sich auf ein Diaphragmalyse-Verfahren nach dem Oberbegriff des Anspruchs 1.

Die Diaphragmaanalyse stellt ein bekanntes Wasseraufbereitungsverfahren dar (vgl. EP 1380543 A1, EP 1382573 A1, WO2006/040709 A1). Dazu wird eine Elektrolysezelle verwendet, die einen Anodenraum mit der Anode und einen von dem Anodenraum durch ein Diaphragma getrennten Kathodenraum mit der Kathode aufweist.

Dem Anoden- und Kathodenraum wird eine wässrige Natriumchlorid-Lösung als Elektrolyt zugeführt. Damit bewegen sich die Anionen und sonstige negativ geladene Teilchen in dem Elektrolyt in Richtung der Anode und die Kationen und dergleichen positiv geladene Teilchen in Richtung Kathode, wobei die negativ geladenen Teilchen in dem Kathodenraum bzw. die positiv geladenen Teilchen in dem Anodenraum das Diaphragma passieren müssen, um zu der Anode bzw. Kathode zu gelangen.

Dadurch entsteht in dem Anodenraum eine Anolyt-Lösung, die ein positives Redox-Potential und damit oxidierende Eigenschaften besitzt, während die in dem Kathodenraum gebildete Katholyt-Lösung ein negatives Redox-Potential, also reduzierende Eigenschaften aufweist. Zudem besitzt die Katholyt-Lösung stark basische Eigenschaften, während die Anolyt-Lösung sauer ist.

Während die Anolyt-Lösung, die Natriumhypochlorid, Wasserstoffperoxid, Chlor und Ozon als metastabile Verbindungen enthält, dem zu reinigenden Wasser zudosiert wird, wird die Katholyt-Lösung, die bisher in wesentlich geringerer Menge anfällt als die Anolyt-Lösung, entweder verworfen oder zur Fällung der Schwermetalle eingesetzt (EP 1380543 A1).

Aufgabe der Erfindung ist es, sowohl für die Katholyt-Lösung wie die Anolyt-Lösung neue Anwendungsgebiete zu erschließen.

Demgemäss wird erfindungsgemäß einerseits ein Verfahren bereitgestellt, das es ermöglicht, die Katholyt-Lösung in größerer Menge zu gewinnen, und zum anderen ein Verfahren, mit dem die metastabilen oxidierenden Eigenschaften der Anolyt-Lösung stabilisiert werden können, um sie für andere Anwendungsgebiete einsetzen zu können.

Überraschenderweise hat sich herausgestellt, dass sich die Katholyt-Produktion wesentlich erhöhen lässt ,wenn die als Elektrolyt eingesetzte Natriumchlorid-Lösung ein Metallsulfat enthält. Möglicherweise wird damit der Transport der Kationen aus dem Anodenraum durch das Diaphragma in den Kathodenraum beschleunigt. Durch die hohe Katholyt-Produktion ergeben sich erfindungsgemäß eine Vielzahl neuer Anwendungsmöglichkeiten für die Katholyt-Lösung.

Dabei wird für das erfindungsgemäße Verfahren beispielsweise ein im Handel erhältliches Diaphragmalyse-Gerät zur Waseraufbereitung verwendet, bei dem der Durchflussregler auf der Kathodenseite zur Erhöhung der Durchflussmenge des Katholyt umgebaut ist.

Neben der gesteigerten Katholyt-Produktion hat sich ferner überraschenderweise gezeigt, dass durch den Zusatz des Metallsulfats zu dem Elektrolyt aus den Sulfat-Anionen in der Anolyt-Lösung weitere metastabile, hochwirksame Verbindungen gebildet werden.

Der Elektrolyt besteht vorzugsweise aus Natriumchlorid, er kann jedoch auch aus einem anderen Alkalichlorid gebildet werden. So hat sich insbesondere ein Gemisch aus Natrium- und Kaliumchlorid als geeignet erwiesen, wobei der Kaliumchloridanteil bis zu 50 Gew.%, bezogen auf das Gesamtgewicht der Alkalichloride betragen kann. Die Alkalichlorid-Lösung wird vorzugsweise als gesättigte Lösung aus einem Vorratsbehälter entnommen und mit der gewünschten Menge Wasser, z. B. in einem Volumenverhältnis von 1:5 bis 1:20 verdünnt. Das Natriumsulfat wird vorzugsweise zu der gesättigten Alkalichlorid-Lösung in dem Vorratsbehälter gegeben. Damit das Metallsulfat in Lösung gehalten wird, kann der Vorratsbehälter erwärmt werden. Das Metallsulfat wird der Alkalichlorid-Lösung vorzugsweise in einer Menge von 1 bis 50 Gramm, insbesondere 5 bis 20 Gramm/Liter zugesetzt.

Das Wasser, mit dem die Alkalichlorid-Lösung verdünnt wird, ist vorzugsweise ein carbonatfreies oder carbonatarmes Wasser, d. h. der (deutsche) Härtegrad des Wassers beträgt vorzugsweise höchstens 5, also der Calciumcarbonatgehalt höchstens 50 mg/l.

Das Metallsulfat, das der Alkalichlorid-Lösung zugesetzt wird, ist vorzugsweise ein Alkalisulfat, insbesondere Natriumsulfat, oder ein Erdalkalisulfat, insbesondere Magnesiumsulfat.

Die Anolyt-Lösung kann eine Redox-Spannung von über +1200 mV und einen pH-Wert von 1 bis 3 aufweisen, die Katholyt-Lösung eine Redox-Spannung von -600 bis -1000 mV und einen pH-Wert von über 12.

Das zweite Ziel der Erfindung, eine stabile Anolyt-Lösung zur Verfügung zu stellen, um für die Anolyt-Lösung neue Anwendungsgebiete erschließen zu können, wird vorzugsweise dadurch erreicht, dass der Anolyt-Lösung eine Metallsulfat zugesetzt wird. Die Menge des zugesetzten Metallsulfats kann 0,1 bis 20 Gramm, insbesondere 0,5 bis 5 Gramm, ganz besonders bevorzugt 1 bis 2 Gramm pro Liter Anolyt-Lösung betragen.

Überraschenderweise hat sich gezeigt, dass damit die metastabilen Verbindungen in der Anolyt-Lösung und damit deren Wirksamkeit über Monate derart zuverlässig stabilisiert werden, dass von einer Lagerstabilität der Anolyt-Lösung von 1 bis 2 Jahren ausgegangen werden kann.

Als Metallsulfat zur Stabilisierung der Anolyt-Lösung wird vorzugsweise Aluminiumsulfat verwendet, insbesondere in Form von Kaliumalaun.

Kaliumalaun, also Kaliumaluminiumsulfat ist in der Naturmedizin als Wirkstoff seit langem bekannt. Es findet z. B. Anwendung im Bereich der Magen-, Darm- und Blasenschwäche, aber auch bei Blutungen. Es ist bekannt, dass Alaun desinfizierend auf Schleimhäute und Wunden wirkt. Gleichzeitig wirkt Alaun allerdings austrocknend, teilweise gerinnend und sekretionsbeschränkend, sodass sein Einsatz in der Medizin eingeschränkt ist.

Es hat sich nun überraschenderweise herausgestellt, dass eine Anolyt-Lösung, die Alaun enthält, eine hervorragende reinigende, dekontaminierende und desinfizierende Wirkung im Mund- und Rachenbereich, ohne nachteilige Folgen auf Zähne, Zahnfüllungen und Zunge, aber auch unter Vermeidung einer Sekretionshemmung und Austrocknung entfaltet. Die erfindungsgemäße, alaunhaltige Anolyt-Lösung ist daher hervorragend als Mundspüllösung geeignet. So können mit ihr auch hartnäckigste Entzündungen im Mund- und Rachenbereich erfolgreich behandelt werden. Blutungen, die z. B. bei der zahnärztlichen Behandlung auftreten, werden innerhalb weniger Minuten gestoppt. Weiterhin kann die Mundspüllösung prophylaktisch eingesetzt werden, um die Entstehung von Parodontopathien, Gingivitis und Stomatitis zu vermeiden.

Die Mundspüllösung kann weiterhin Magnesiumsulfat enthalten, wodurch die Lagerstabilität weiter erhöht wird. Die Menge des Magnesiumsulfats kann beispielsweise 0,5 bis 10 Gramm pro Liter Anolyt-Lösung, insbesondere 1 bis 2 Gramm pro Liter betragen.

Die Mundspüllösung kann weitere allgemein übliche Zusatzstoffe enthalten, beispielsweise Aromastoffe, Süssstoffe, ätherische Öle und dergleichen.

Es hat sich gezeigt, dass die erfindungsgemäße Mundspüllösung Keime, die für das Entstehen von Plaque, Gingivitis und Parodontitis verantwortlich sind, zuverlässig beseitigt. Infektionen werden so wirksam vorgebeugt und eventuell vorhandene Wunden heilen beschleunigt ab. Ebenso wenig treten Verfärbungen des Zahnfleisches, der Zähne oder der Zunge auf.

Die erfindungsgemäße Anolyt-Lösung ist jedoch nicht nur als Mundspüllösung verwendbar, vielmehr ist sie auf den verschiedensten Indikationsgebieten einsetzbar und zwar sowohl in der Human- wie in der Tiermedizin. So besitzt sie eine hohe biozide, insbesondere antibakterielle und fungizide Wirkung.

So kann die erfindungsgemäße Anolyt-Lösung zur Wundbehandlung, beispielsweise bei Hautverletzungen oder zur Behandlung von Geschwüren eingesetzt werden. Insbesondere hat sich die erfindungsgemäße Anolyt-Lösung hervorragend zur Behandlung des Magen-Darm-Trakts erwiesen, um beispielsweise durch Heliobacter oder Candida hervorgerufene Krankheiten oder Blutungen zu beseitigen. Neben einer starken antibakteriellen Wirkung weist die erfindungsgemäße Anolyt-Lösung zudem eine stark fungizide Wirkung auf. Damit können sowohl beim Menschen Pilzinfektionen, beispielsweise Nagelpilz erfolgreich bekämpft werden, wie z. B. die Huffäule, Mauke oder der Sattelpilz bei Pferden.

Darüber hinaus kann die erfindungsgemäße Anolyt-Lösung zur Behandlung von Ekzemen, beispielsweise dem Sommerekzem bei Pferden eingesetzt werden, aber auch zur Bekämpfung der Stirnhöhlenvereiterung z. B. bei Pferden, sowie von Entzündungen und Infektionen bei Tier und Mensch, bis hin zur Zeckenprophylaxe. Dabei ist die erfindungsgemäße Anolyt-Lösung insbesondere auch bei Krankheiten einsetzbar, die aufgrund von Antibiotikaresistenzen nicht mehr behandelt werden können.

Ein weiteres Anwendungsgebiet der erfindungsgemäßen Analyt-Lösung stellt die Haltbarmachung von Cremes, beispielsweise zur Körperpflege dar. Denn neben der desinfizierenden Wirkung weist die erfindungsgemäße Anolyt-Lösung zugleich eine stark emulgierende Wirkung auf. Zudem hat sie gegenüber anderen in Cremes verwendeten Zusatzstoffen den Vorteil, dass sie zu keinen Allergien führt.

Weiterhin hat sich herausgestellt, dass sich die erfindungsgemäße Anolyt-Lösung hervorragend als Trinkwasserzusatz bei der Geflügelhaltung eignet.

So wurden letztes Jahr, als aufgrund der Vogelgrippe die Hühner aufgestallt werden mussten, in einer Hühnerfarm mit mehreren 100 Hühnern, die eine Hälfte mit Trinkwasser versorgt, welches die erfindungsgemäße Anolyt-Lösung in einer Verdünnung von 1 Volumenteil Anolyt-Lösung zu zehn Volumenteilen Wasser in der ersten Woche und in einer Verdünnung von 1:100 in den darauf folgenden fünf Wochen enthielt, während die andere Hälfte der Hühner als Kontrollgruppe das Trinkwasser ohne die Anolyt-Lösung verabreicht bekam. Nach sechs Wochen wurde eine Gewichtszunahme bei der Hühnern, die die Anolyt-Lösung erhielten, von über 30 % gegenüber der Kontrollgruppe festgestellt.

Außer zur Geflügelhaltung ist die Anolyt-Lösung generell in der Tierzucht als Trinkwasserzusatz geeignet.

Zudem ist die erfindungsgemäße Anolyt-Lösung hervorragend als Reinigungsmittel z.B. im Lebensmittelbereich geeignet. So kann sie beispielsweise zur Reinigung von Leitungen, Behältern oder dergleichen z.B. in Brauereien oder Molkereien oder generell zur Reinigung von Anlagen zur Nahrungsmittel- einschließlich Getränkeproduktion eingesetzt werden. Dazu gehört insbesondere auch die Herstellung Kühleis. So ist Kühleis, das mit einer mit der erfindungsgemäßen Anolyt-Lösung gereinigte Anlage hergestellt worden ist, aufgrund seiner Keimfreiheit in direktem Kontakt auch mit leicht verderblichen Lebensmitteln, wie Fischen, mit großen Erfolg eingesetzt worden.

Das heißt, die erfindungsgemäß hergestellte Anolyt-Lösung ist generell zur Reinigung von Oberflächen im Nahrungsmittelbereich, einschließlich Getränke, verwendbar. Insbesondere kann sie zur Reinigung von Anlagen oder Gegenständen, die zur Herstellung von Nahrungsmitteln eingesetzt werden, oder von Hilfsmitteln, wie Kühleis, die mit Nahrungsmitteln in Kontakt kommen, verwendet werden.

Dabei kann insbesondere eine Kombination aus der erfindungsgemäße Anolyt-Lösung und der erfindungsgemäßen Katholyt-Lösung zur Anwendung kommen. Dies gilt nicht nur für den Lebensmittelbereich, sondern auch für andere Reinigungsanwendungen. Dabei wird vorzugsweise erst mit der erfindungsgemäßen, stark basischen Katholyt-Lösung und anschließend mit der erfindungsgemäßen Anolyt-Lösung gereinigt.

Auch ist die erfindungsgemäße Katholyt-Lösung zur Entkeimung und Desinfektion von Oberflächen hervorragend geeignet. So kann die erfindungsgemäße Katholyt-Lösung beispielsweise zur Oberflächenreinigung in Arztpraxen, Krankenhäusern und dergleichen Gesundheitseinrichtungen oder Altersheimen eingesetzt werden. Aufgrund des hohen pH-Wertes der Katholyt-Lösung wird dabei auch der für diese Einrichtungen oft spezifische unangenehme Geruch beseitigt. Dabei kann sich an die Oberflächenbehandlung mit der Katholyt-Lösung eine Behandlung mit der erfindungsgemäßen Anolyt-Lösung anschließen, um die Entkeimung und Desinfektion zu vervollständigen. Die erfindungsgemäße Anolyt-Lösung besitzt eine hohe reinigende Wirkung und ist daher generell zur Herstellung von Reinigungsmitteln verwendbar.

Außerdem kann die erfindungsgemäße Katholyt-Lösung aufgrund ihrer hohen Alkalität beispielsweise zur pH-Wert-Neutralisierung saurer Böden eingesetzt werden. So kann beispielsweise das Pilzwachstum im Boden durch eine pH-Wert-Verschiebung vom sauren in den neutralen Bereich unterdrückt werden, wodurch das Wachstum der Pflanzen durch die erhöhte Nährstoffaufnahme spürbar verbessert werden kann.

Als weiteres Anwendungsgebiet der erfindungsgemäßen Katholyt-Lösung ist die Reinigung von stark mit Schwermetallen belastetem Wasser, beispielsweise dem Wasser von Textilfärbereien, Brandwasser oder dem Abwasser von Kaltwalzwerken, zu nennen. Der durch die erfindungsgemäße Katholyt-Lösung beispielsweise auf 9 bis 10 erhöhte pH-Wert des Wassers führt zu einer praktisch quantitativen Ausfällung der Schwermetalle. Die gebildeten Flocken können mit einem Gesteinsmehl, beispielsweise Basaltstaub, gebunden und damit z. B. durch Filtrieren mit einem aus Sintermetall gebildeten Filter abgetrennt werden. Das aus dem Filtrat gebildete Restwasser kann beispielsweise mit der erfindungsgemäßen Anolyt-Lösung nachgereinigt werden.

## Patentansprüche

1. Verwendung einer Katholyt-Lösung, die in einem Diaphragmalyse-Verfahren erhalten wird, bei dem dem Anodenraum und dem Kathodenraum einer Elektrolysezelle, die durch ein Diaphragma voneinander getrennt sind, jeweils eine wässrige Alkalichlorid-Lösung zugeführt wird zur Entkeimung und/oder Desinfektion von Oberflächen.

2. Verwendung nach Anspruch 1, **dadurch gekennzeichnet, dass** die Anolyt-Lösung, die mit dem Diaphragmalyse-Verfahren erhalten wird nach der Oberflächendesinfektion mit der Katholyt-Lösung eingesetzt wird.

3. Verwendung nach Anspruch 1, **dadurch gekennzeichnet, dass** die erhaltene Katholyt-Lösung zur Behandlung des Bodens eingesetzt wird.

4. Verwendung nach Anspruch 1, **dadurch gekennzeichnet, dass** die erhaltene Katholyt-Lösung zur Aufbereitung von mit Schwermetallen verunreinigtem Wasser durch Ausfällung der Schwermetalle eingesetzt wird.

5. Verwendung nach Anspruch 4, **dadurch gekennzeichnet, dass** die mit der erhaltene Katholyt-Lösung ausgefällten Schwermetalle mit einem Gesteinsmehl gebunden und abgetrennt werden.

6. Verwendung nach Anspruch 2 oder 3, **dadurch gekennzeichnet, dass** eine Anolyt-Lösung eingesetzt wird, die nach einem Diaphragmalyse-Verfahren erhalten wird, bei dem dem Anodenraum und dem Kathodenraum einer Elektrolysezelle, die durch ein Diaphragma voneinander getrennt sind, jeweils eine wässrige Alkalichlorid-Lösung zugeführt wird, um eine Anolyt-Lösung und eine Katholyt-Lösung zu bilden und bei dem der zugeführten Alkalichlorid-Lösung zur Erhöhung der Katholyt-Produktion ein Metallsulfat zugesetzt wird.

7. Verwendung nach Anspruch 2 oder 3, **dadurch gekennzeichnet, dass** zum Erhalt der Anolyt-Lösung der zugeführten Alkalichlorid-Lösung 1 bis 50 Gramm Metallsulfat pro Liter zugesetzt werden.

8. Verwendung nach Anspruch 3 oder 4, **dadurch gekennzeichnet, dass** das zum Erhalt der Anolyt-Lösung zugesetzte Metallsulfat ein Alkali- und/oder Erdalkalisulfat ist.

9. Verwendung nach Anspruch 2 oder 3, **dadurch gekennzeichnet, dass** das zum Erhalt der Anolyt-Lösung zugesetzte Alkalisulfat Natriumsulfat bzw. das Erdalkalisulfat Magnesiumsulfat ist.

10. Verwendung nach Anspruch 2 oder 3, **dadurch gekennzeichnet, dass** die zum Erhalt der Anolyt-Lösung zugeführte wässrige Alkalichlorid-Lösung weniger als 50 mg Calciumcarbonat pro Liter enthält.

11. Verwendung nach Anspruch 2, **dadurch gekennzeichnet, dass** der Anolyt-Lösung ein Metallsulfat zugesetzt wird.

12. Verwendung nach Anspruch 8, **dadurch gekennzeichnet, dass** die Menge des zugesetzten Metallsulfats 0,1 bis 20 Gramm/Liter beträgt.

13. Verwendung nach Anspruch 8 oder 9, **dadurch gekennzeichnet, dass** das Metallsulfat Aluminium- und/oder Magnesiumsulfat ist.

14. Verwendung nach Anspruch 10, **dadurch gekennzeichnet, dass** als Aluminiumsulfat Alaun verwendet wird.

15. Verwendung nach einem oder mehreren der vorhergehenden Anspruche, **dadurch gekennzeichnet, dass** der zugeführten Alkalichlorid-Lösung zur Erhöhung der Katholyt-Produktion 1 bis 50 Gramm Alkalisulfat pro Liter zugesetzt werden und der Anolyt-Lösung Aluminium und/oder Magnesiumsulfat in einer Menge von 0,1 bis 20 Gramm/Liter zugesetzt wird.
